(19) Europäisches Patentamt
European Patent Office
Office européen des brevets

(11) Publication number: **0 614 661 A2**

# (12) EUROPEAN PATENT APPLICATION

(21) Application number: **93119976.4**

(22) Date of filing: **21.12.88**

(51) Int. Cl.[5]: **A61K 31/165**, A61K 31/275, C07C 255/36, C07C 255/37, C07C 255/40, C07C 255/43, C07C 255/41, A61K 31/19, A61K 31/36, C07D 317/62

This application was filed on 10 - 12 - 1993 as a divisional application to the application mentioned under INID code 60.

(30) Priority: **24.12.87 IL 8493787**
**10.11.88 IL 8835488**

(43) Date of publication of application:
**14.09.94 Bulletin 94/37**

(60) Publication number of the earlier application in accordance with Art.76 EPC: **0 322 738**

(84) Designated Contracting States:
**AT BE CH DE ES FR GB GR IT LI LU NL SE**

(71) Applicant: **YISSUM RESEARCH DEVELOPMENT COMPANY OF THE HEBREW UNIVERSITY OF JERUSALEM**
**46 Jabotinsky Street**
**Jerusalem, 92 182 (IL)**

(72) Inventor: **Levitzki, Alexander**
**36 Palmach Street**
**Jerusalem (IL)**
Inventor: **Gilon, Chaim**
**18 Gelbert Street**
**Jerusalem (IL)**
Inventor: **Chorev, Michael**
**35 Feinstein Street**
**Talpiot Mizrach, Jerusalem (IL)**
Inventor: **Gazit, Aviv**
**14 Nof Harim Street**
**Jerusalem (IL)**

(74) Representative: **Brown, John David**
**FORRESTER & BOEHMERT**
**Franz-Joseph-Strasse 38**
**D-80801 München (DE)**

(54) **Benzylidene- and cinnamylidine-malononitrile derivatives for the inhibition of proliferative processes in mammalian cells.**

(57) There are provided pharmaceutical compositions containing as an active ingredient a compound of the general formula (I):

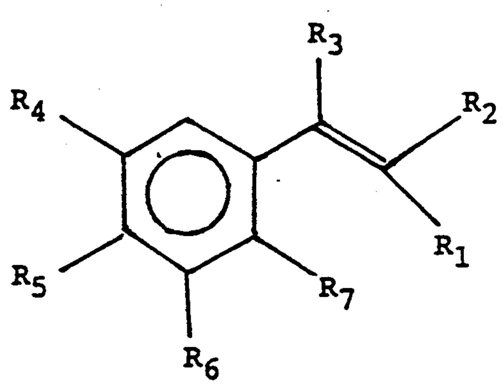

(I)

EP 0 614 661 A2

wherein $R_1$ and $R_2$ are each independently CN, $CONH_2$ or COOH or one of $R_1$ ad $R_2$ may be $-CSNH_2$ or, when $R_1$ is CN, $R_2$ can also be the group

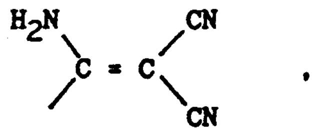

,

$R_3$ is H, $CH_3$ or OH,

$R_4$, $R_5$, $R_6$, $R_7$ are each independently H, OH, $C_{1-5}$ alkyl, $C_{1-5}$ alkoxy, $NH_2$, CHO, halogen, $NO_2$ or COOH, or $R_4$ and $R_5$ together may represent a group $-O-CH_2-O-$;

provided that: (a) when $R_4$ ad $R_7$ are each OH, $R_3$, $R_5$ and $R_6$ are each H and one of $R_1$ and $R_2$ is CN, then the other of $R_1$ and $R_2$ cannot be $CONH_2$; and (b) when $R_3$ and $R_7$ are each H, $R_5$ is OH and $R_4$ and $R_6$ are both H or both $C_{1-5}$ alkyl, then $R_1$ is CN and $R_2$ is CN or the group

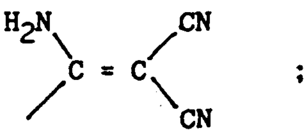

;

or a pharmaceutically acceptable salt thereof.

There are also provided some novel compounds of formula (I) above.

The compositions and compounds according to the invention are efficient protein-tyrosine kinase inhibitors and are suitable for the inhibition of proliferative processes in mammalian cells.

FIGURE 1

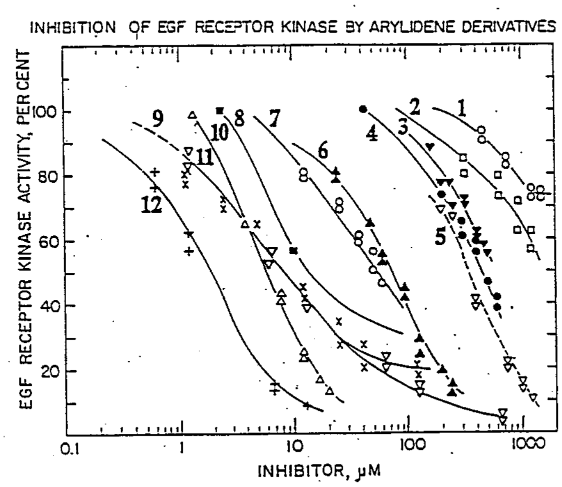


| COMPOUND | SUBSTITUENTS | | | | | | | |
|---|---|---|---|---|---|---|---|---|
| | $R_3$ | $R_4$ | $R_5$ | $R_6$ | $R_7$ | $R_2$ | $R_1$ | KInh μM |
| 1 | H | H | OH | H | H | $CO_2H$ | H | 1000 |
| 2 | H | H | OH | H | H | $CO_2H$ | $CO_2H$ | 500 |
| 3 | H | H | OH | H | H | CN | CN | 166 |
| 4 | H | OH | OH | H | H | $CO_2H$ | H | 150 |
| 5 | H | H | H | OH | H | CN | CN | 123 |
| 6 | H | OH | H | H | OH | CN | $CO_2H$ | 24 |
| 7 | H | H | OH | OH | H | $CO_2H$ | CN | 18 |
| 8 | H | H | OH | OH | H | CN | CN | 11 |
| 9 | H | $OCH_3$ | OH | OH | H | CN | CN | 2 |
| 10 | H | OH | OH | OH | H | CN | CN | 1 |
| 11 | H | H | OH | OH | H | $CONH_2$ | CN | 2.3 |
| 12 | H | H | OH | OH | H | $CSNH_2$ | CN | 0.85 |

## FIELD OF INVENTION

The present invention concerns novel pharmaceutical compositions containing substituted benzylidene- and cinnamylidene-malononic acid derivatives for specifically inhibiting cell proliferation processes in mammals. The invention further provides certain novel compounds of the aforesaid type.

## BACKGROUND OF THE INVENTION

Currently the chemotherapy of cancer makes use of inhibitors of DNA synthesis (examples: adriamycin, fluorouracil) and compounds which disrupt the cytoskeleton (vinblastine). These compounds are highly toxic since their inhibitory activity is not limited to cancer cells, with the distinction, however, that tumor cells are more readily attacked by the aforesaid inhibitors because these cells divide more rapidly and their DNA metabolism is consequently more active. A few types of cancers are nowadays treated with specific pharmaceutical agents. These include, for example, certain breast cancers which are hormone dependent and can therefore be treated with specific hormone derivatives. These cases, however, are the exception and the chemotherapeutic treatment for the majority of the various types of cancer is non-specific.

In the early 1980's it became apparent that 20 to 30 percent of cancers express characteristic oncogenic products which are growth factor receptors or their mutated homologs, which exhibit protein tyrosine kinase (PTK) activity. The PTK activity is intrinsic to the receptor or its oncogene homolog and, which influences the cell proliferation via its PTK domain. Furthermore, each of these receptors (normal or mutated), exhibits a characteristic PTK activity with a distinct substrate specificity. One of these receptors is the epidermal growth factor (EGF) receptor and its oncogenic homolog v-Erb-b. Pursuant to that discovery it has already been proposed to treat cancer by means of various chemical substances capable of inhibiting the PTK activity of EGF - see, for example, in Japanese patents Nos. 6239523, 6242923 and 6242925.

It is the object of the present invention to provide readily accessible compounds of relatively simple structure that are active as specific EGFR-tyrosine kinase inhibitors and can thus serve as specific anticancer agents.

## GENERAL DESCRIPTION OF THE INVENTION

The above object is achieved by the present invention which provides pharmaceutical compositions containing as an active ingredient a compound of the general formula (I):

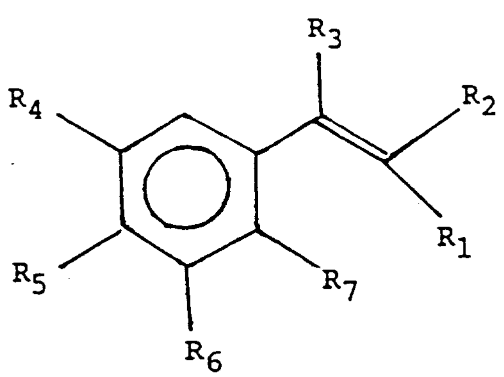

(I)

wherein $R_1$ and $R_2$ are each independently CN, $CONH_2$ or COOH or one of $R_1$ and $R_2$ may be $-CSNH_2$ or, when $R_1$ is CN, $R_2$ can also be the group

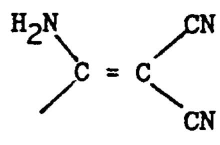

,

$R_3$ is H, $CH_3$ or OH,

$R_4$, $R_5$, $R_6$, $R_7$ are each independently H, OH, $C_{1-5}$ alkyl, $C_{1-5}$ alkoxy, $NH_2$, CHO, halogen, $NO_2$ or COOH,

or $R_4$ and $R_5$ together may represent a group $-O-CH_2-O-$;
provided that: (a) when $R_4$ and $R_7$ are each OH, $R_3$, $R_5$ and $R_6$ are each H and one of $R_1$ and $R_2$ is CN, then the other of $R_1$ and $R_2$ cannot be $CONH_2$; and (b) when $R_3$ and $R_7$ are each H, $R_5$ is OH ad $R_4$ and $R_6$ are both H or both $C_{1-5}$ alkyl, then $R_1$ is CN and $R_2$ is CN or the group

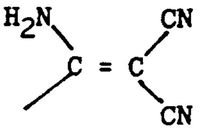 ;

or a pharmaceutically acceptable salt thereof.

Preferred pharmaceutical compositions are those comprising an active ingredient of formula I in which at least one of $R_1$ and $R_2$ is CN cis to the phenyl moiety of said formula.

Amongst the preferred compositions, more preferred are those comprising an active ingredient in which $R_4$ and $R_5$ are hydroxy groups, $R_6$ is hydrogen or hydroxy and $R_3$ and $R_7$ are hydrogens.

Especially preferred pharmaceutical compositions are those containing as an active ingredient a compound selected from:
3,5-dihydroxybenzylidene-malononitrile,
α-hydroxy-3,4,5-trihydroxybenzylidene-malononitrile,
3-methoxy-4,5-dihydroxybenzylidene-malononitrile,
α-cyano-3,4-dihydroxycinnamthioamide,
α-cyano-3,4-dihydroxy-cinnamamide,
3,5-di-t-butyl-4-hydroxybenzylidene-malononitrile,
4-formylbenzylidene-malononitrile,
4-hydroxybenzylidene-malononitrile,
3,4-methylenedioxy-6-nitrobenzylidene-malononitrile,
3,4-dihydroxybenzylidene-malononitrile,
3,4,5-trihydroxybenzylidene-malononitrile,
γ-cyano-β-amino-3,4-dihydroxycinnamylidene-malononitrile,
γ-cyano-β-amino-3,4,5-trihydroxycinnamylidene-malononitrile,
γ-cyano-β-amino-3,4-dihydroxy-5-methoxycinnamylidene-malononitrile,
γ-cyano-β-amino-3,4-dihydroxy-5-bromocinnamylidene-malononitrile, and
γ-cyano-β-amino-3-hydroxy-4-nitrocinnamylidene-malononitrile; and pharmaceutically acceptable salts thereof.

According to another aspect of the invention, there are also provided novel compounds of the formula (I) above, selected from:
3,5-dihydroxybenzylidene-malononitrile,
α-hydroxy-3,4,5-trihydroxybenzylidene-malononitrile,
α-cyano-3,4-dihydroxycinnamthioamide,
4-formylbenzylidene-malononitrile,
3,4-methylenedioxy-6-nitrobenzylidene-malononitrile,
γ-cyano-β-amino-3,4-dihydroxycinnamylidene-malononitrile,
γ-cyano-β-amino-3,4,5-trihydroxycinnamylidene-malononitrile,
γ-cyano-β-amino-3,4-dihydroxy-5-methoxycinnamylidene-malononitrile,
γ-cyano-β-amino-3,4-dihydroxy-5-bromocinnamylidene-malonolnitrile,
γ-cyano-β-amino-3-hydroxy-4-nitrocinnamylidene-malononitrile; and pharmaceutically acceptable salts thereof.

The malonic acid derivatives of formula (I) above, can be prepared by known methods, for example by reacting a corresponding substituted benzaldehyde with malononitrile to obtain the benzylidene derivatives or with malononitrile dimers to obtain the cinnamylidene derivatives. The reaction is generally carried out in a suitable solvent, such as ethanol or benzene, and in the presence of a catalyst, e.g., piperidine, pyridine or β-alanine. Alternatively, a suitably substituted benzoyl chloride, e.g. triacetyl-galloyl chloride, can be reacted with malononitrile in the presence of an amine in a non-polar organic solvent.

The EGFR-inhibitor activity was tested on partially purified EGF receptors and on cell culture samples and the results are summarized in Table 1 herein.

In order to better understand the invention, reference will be made to the attached drawings, in which:

Fig. 1 is a graphical representation of the activity of isolated EGFR kinases (given in percent of the total kinase activity) plotted against the concentrations in μM of 12 different inhibitors.
Figs. 2a and 2b are graphical representations of the inhibitory effect of two pairs of tested compounds on the rate of the growth of KB and A431 cells, respectively, the number of cells being plotted against time (in days).
Fig. 3 is a graphical representation of the inhibition of A 431 cell growth as a function of various concentrations (in μM) of the inhibitor "compound 2" according to the invention.
Figs. 4a and 4b are graphical representations of the inhibitory effect of two pairs of tested compounds on the rate of the EGF dependent proliferation of A431/clone 15 cells. Fig. 4a depicts inhibition effects of compounds found to inhibit EGF dependent growth preferentially and Fig. 4b depicts inhibition effects of compounds found to inhibit EGF dependent growth exclusively.

The invention will now be described in more detail in the following non-limiting examples.

## Preparative Examples

### Example 1

3,4-Dihydroxybenzylidene-malononitrile

(Table 1, Compound 2)

To 11g (80 mM) of 3,4-dihydroxybenzaldehyde and 5.5g (83 mM) of malononitrile in 40 ml of ethanol, 7 drops of piperidine were added. The mixture was then heated at 70°C for 0.5-1 hour and then poured into water. The resulting solid precipitate was separated by filtration to give 12.7g (86% yield) of a yellow solid, 3,4-dihydroxybenzylidene-malononitrile, m.p.225.
Following the same procedure there were prepared:
3,5-dihydroxybenzylidene-malononitrile (Compound 1 in Table 1),
3-methoxy-4,5-dihydroxybenzylidene-malononitrile (Compound 3 in Table 1),
3,4,5-trihydroxybenzylidene-malononitrile (Compound 4 in Table 1).
3,5-di-t-butyl-4-hydroxybenzylidene-malononitrile,
3-hydroxybenzylidene-malononitrile.

### Example 2

α-hydroxy-3,4,5-trihydroxybenzylidene-malononitrile

(Table 1, Compound 5)

To 2g (30mM) of malononitrile and 4 ml (40 mM) of triethylamine in 100 ml of $CH_2Cl_2$, triacetyl galloyl chloride (prepared from 7g (24 mM) of triacetyl gallic acid and thionyl chloride) in 50 ml $CH_2Cl_2$ was added. The resulting mixture was then stirred for 2 hours at room temperature, poured into 50 ml of water and hydrolyzed by heating for 2 minutes at 80°C with a solution of 2.5g of NaOH in 30 ml of ethanol. The mixture was then extracted with ethyl acetate and the organic extract was further worked up by washing with water, drying, filtering and evaporating it. Chromatography on silica gel gave 1.5g (29% yield) of α-hydroxy 3,4,5-trihydroxybenzylidene-malononitrile as an oily solid.

### Example 3

α-cyano-3,4-dihydroxycinnamamide

(Table 1, Compound 6)

Reaction of 2.4g (10 mM) of 3,4-dihydroxybenzaldehyde and 0.9g (10.7 mM) of cyanoacetamide, by the procedure described in Example 1 above, gave 1.45g (70% yield) of α-cyano-3,4-dihydroxycinnamamide, as a yellow solid, m.p.247°C.

**Example 4**

γ-cyano-β-amino-3,4-dihydroxycinnamylidene-malononitrile

(Table 1, Compound 7)

1.4g (10 mM) of 3,4-dihydroxybenzaldehyde, 1.4g (10.6 mM) of malononitrile dimer and 0.3g of β-alanine in 50 ml ethanol were heated at 70°C for 40 minutes. 100 ml of water were added and the suspension was cooled and a solid precipitate was filtered off, washed with water and dried to give 1.3g of a yellow-orange solid, mp.235°C, 53% yield, of γ-cyano-β-amino-3,4-dihydroxycinnamylidene-malononitrile.

Following the same procedure there were prepared:
γ-Cyano-β-amino-3,4,5-trihydroxycinnamylidene-malononitrile (Compound 12 in Table 1), and
γ-Cyano-β-amino-3-hydroxy-4-nitrocinnamylidene-malononitrile (Compound 15 in Table 1).

**Example 5**

α-cyano-3,4-dihydroxycinnamic acid

(Table 1, Compound 8)

a) 2g (15 mM) of 3,4-dihydroxybenzaldehyde, 3g (21 mM) of t-butyl cyanoacetate and 0.5 ml of piperidine in 50 ml ethanol were heated to reflux for 1 hour. The resulting mixture was then poured into water and a solid precipitate was separated by filtration and was then washed and dried to yield 2.5g (yield 66%) of t-butyl α-cyano-3,4-dihydroxycinnamate as a yellow solid.

b) 1.6g of the t-butyl ester from a) in 10 ml of Trifluoro Acetic Acid was stirred at room temperature for 20 minutes. 50 ml of $H_2O$ were added and the cooled suspension filtered to yield a solid precipitate which was washed with water and dried to give 1g (yield 85%) of α-cyano-3,4-dihydroxycinnamic acid as a yellow solid, mp.240°C.

**Example 6**

α-cyano-3,4-dihydroxycinnamthioamide

(Table 1, Compound 9)

To 0.83g (6 mM) 3,4-dihydroxybenzaldehyde and 0.7g (7 mM) cyanothioacetamide in 30 ml ethanol were added 4 drops of piperidine. The mixture was refluxed for 1 hour and poured into ice-water. Filtering and drying gave 0.54 g, (41% yield), of an orange solid, mp.213°C.

| | | | |
|---|---|---|---|
| Anal. Calc. for $C_{10}H_8N_2O_2S$: | C = 54.54, | H = 3.64, | N = 12.73; |
| Found: | C = 54.44, | H = 3.87, | N = 12.91 |

MS: 220(M+,93%), 219(M-1,100%), 203(M-OH,26%), 186(M-2OH,24%), 123(33%), 110(30%), 100(43%), m/e.

Example 7

3,4-methylenedioxy-6-nitrobenzylidene malononitrile

(Table 1, Compound 11)

1g (5.1 mM) 3,4-methylenedioxy-6-nitrobenzaldehyde, 0.4 g (6 mM) malononitrile and 0.2 g β-alanine in 30 ml ethanol were stirred 16 hours at room temperature. 50 ml $H_2O$ were added. Filtering gave 1g, (80% yield) of a bright yellow solid, mp.104°C.
NMR (acetone-$d_6$): 6.42 (2H, s, methylenedioxy), 7.45 (1H, s, $H_2$), 7.82 (1H, s, $H_5$), 8.70 (1H, s, vinylic proton).
Following the same procedure there were also prepared:

γ-Cyano-β-amino-3,4-dihydroxy-5-methoxycinnamylidene-malononitrile (Compound 13 in Table 1), and
γ-Cyano-β-amino-3,4-dihydroxy-5-bromocinnamylidene-malononitrile (Compound 14 in Table 1).

In Table 1 below there are listed 15 compounds according to the invention, 11 of which are new. All compounds gave correct analytical and spectroscopic data.

In this Table, KInh is the dissociation constant of the complex PTK-inhibitor and is expressed in μM units. The different KInh values were determined by the analysis according to Dixon.

**Table 1**

| No. | | mp, °C | KInh, µM | Literature |
|---|---|---|---|---|
| 1 | 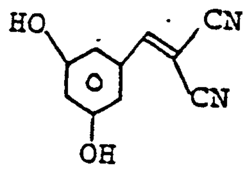 | 175 | 10 | New |
| 2 | 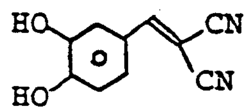 | 225 | 11±0.1 | 1* |
| 3 | 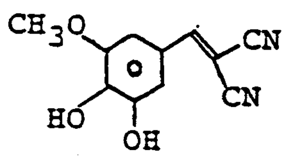 | 235 | 2.2±0.3 | New |
| 4 | 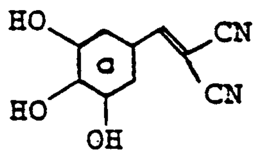 | 245 | 1 | 1* |
| 5 | 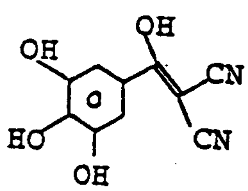 | oil | 4.5 | New |
| 6 | 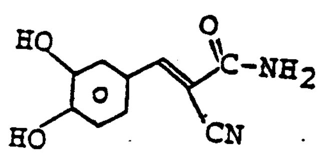 | 247 | 3.5±0.6 | 1* |
| 7 | 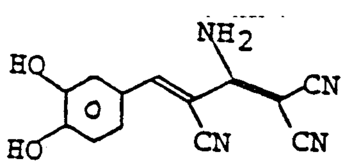 | 235 | Non-competitive inhibitor | New |

Table 1 (continued)

| No. | | mp, °C | KInh, μM | Literature |
|---|---|---|---|---|
| 8 | 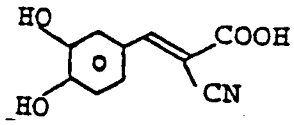 | 240 | 23.6 | 1* |
| 9. | 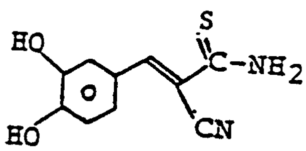 | 213 | 0.85 | New |
| 10. | 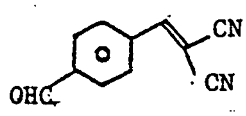 | 142 | 20 | New |
| 11. | 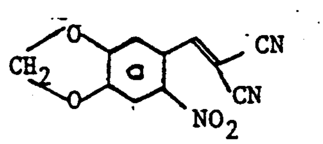 | 104 | - ** | New |
| 12. | 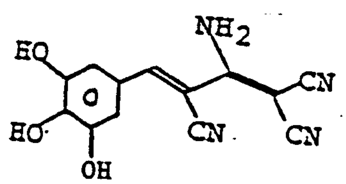 | 275 | Non-competitive inhibitor | New |
| 13. | 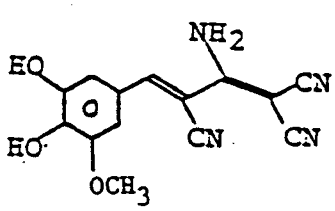 | 225 | Non-competitive inhibitor | New |
| 14. | 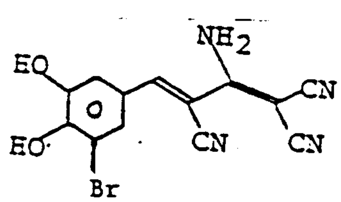 | 241 | Non-competitive inhibitor | New |

Table 1 (continued)

| No. | | mp,°C | KInh,µM | Literature |
|---|---|---|---|---|
| 15. | 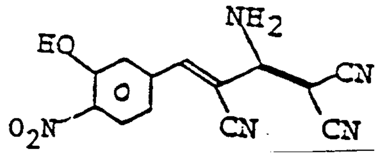 | 219 | Non-competitive inhibitor | New |

* K.W. Rosemund and T. Boehm, ann. 437, 125 (1924).
** KInh was not calculated.

## EGFR Inhibition Tests

Test on extracted EGF Receptors:

EGF receptors were prepared from A431 cells (obtained from the ATCC) and PTK activity of these receptors was assayed as described by S. Braun, W. E. Raymond and E. Racker, J. Biol. Chem. 259, 2051-2054 (1984). The compounds listed in Table 1 were tested for their inhibitory capacity on the EGF-receptor kinase activity, using the assay described above. Figure 1 demonstrates characteristic results using 10 inhibitors. The assay conditions were as described above using 0.125 mg of copoly $Glu^6Ala^3Tyr^1$. Dissociation constants were calculated from the inhibition curves and are listed in Table 1 above and indicated for each formula in Figure 1.

Tests on cells in tissue culture:

a) A431 cells and KB cells express EGF receptors on their cell surface and their growth rate depends on the presence of growth factors in the medium.
These cells were seeded and grown as described in O.Kashles and A.Levitzki, Bichem. Pharmacol., 35, 1531-1536 (1987). The compounds, the formulae of which are given in Fig. 2, were added to the medium at a cells concentration of Ca. $2x10^5$ cells/well. The inhibitor was added to the medium 1 hour after seeding. The medium volume in a well was 1 ml and the concentration of inhibitor therein 20 µM. Every 24 hours cells were counted and fresh medium with inhibitor applied to the remaining wells. The growth curves were determined in 24-well Costar dishes.
b) Some of the compounds according to the present invention are exclusive inhibitors to EGF dependent growth of cells and others are preferential inhibitors to such growth. Examples of the former are depicted in Fig. 4b and of the latter in Fig. 4a. In the experiment depicted in these Figures, 25,000 cells per well were placed in a 24 wells plate (Costar) supplied with Dulbeco medium containing 10% foetal calf serum, with 10 ng/ml EGF(●, ■, ▲) or with no added EGF (○, □ ,△). EGF receptor kinase inhibitors at various concentrations were added to the cells two hours after plating. The medium containing the inhibitors was replaced with fresh inhibitor containing medium every other day. On the fifth day, the number of cells in the presence of EGF and in the absence of EGF was determined. In Figs. 4a and 4b "100%" refers to the number of cells in the absence of inhibitor for each mode of cell growth (without EGF: 100,000 ± 10,000 cells; with EGF: 260,000 ± 30,000 cells for seven experiments). The filled symbols (●, ■, ▲) in Figs. 4a and 4b refer to inhibition of EGF stimulated growth, whereas open symbols (○, □, △) depict inhibition of EGF independent growth. Each experimental point represents the average of triplicate determination where the variance was less than 5 per cent. The compound numbers refer to compounds in Fig. 1.

The features disclosed in the foregoing description, in the claims and/or in the accompanying drawings may, both separately and in any combination thereof, be material for realising the invention in diverse forms thereof.

**Claims**

**1.** Pharmaceutical compositions containing as an active ingredient a compound of the general formula (I):

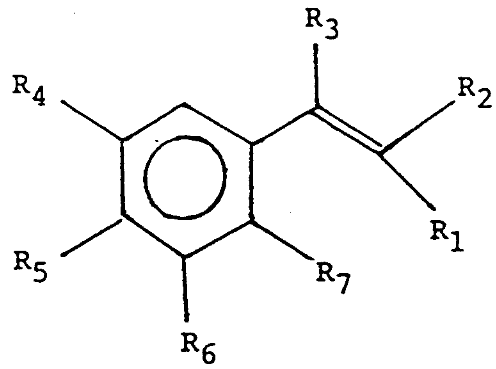

(I)

wherein one of $R_1$ and $R_2$ is CN and the other of $R_1$ and $R_2$ is $-C(X)NH_2$, in which X is O or S,
$R_3$ is H, $CH_3$ or OH,
$R_4$, $R_5$, $R_6$, $R_7$, are each independently H, OH, $C_{1-5}$ alkyl, $C_{1-5}$ alkoxy, $NH_2$, CHO, halogen, $NO_2$ or COOH, or $R_4$ and $R_5$ together may represent a group $-O-CH_2-O-$;
provided that: (a) when $R_4$ and $R_7$ are each OH, $R_3$, $R_5$ and $R_6$ are each H and one of $R_1$ and $R_2$ is CN, then the other of $R_1$ and $R_2$ cannot be $CONH_2$; and (b) when $R_3$ and $R_7$ are each H, $R_5$ is OH and $R_4$ and $R_6$ are both H or both $C_{1-5}$ alkyl, then $R_1$ is CN and $R_2$ is CN or the group

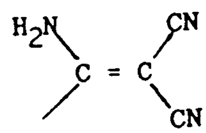

;

or a pharmaceutically acceptable salt thereof.

**2.** The use of a compound of the general formula I, or a pharmaceutically acceptable salt thereof, for the preparation of a medicament.

**3.** Pharmaceutical compositions containing as an active ingredient a compound of the general formula (I):

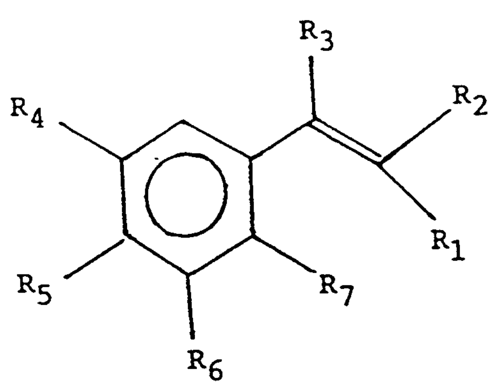

(I)

wherein $R_1$ and $R_2$ are each independently CN, $CONH_2$ or COOH or one of $R_1$ and $R_2$ may be $-CSNH_2$ or, when $R_1$ is CN, $R_2$ can also be the group

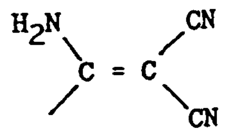

$R_3$ is H, $CH_3$ or OH,
$R_4$, $R_5$, $R_6$, $R_7$ are each independently H, OH, $C_{1-5}$ alkyl, $C_{1-5}$ alkoxy, $NH_2$, CHO, halogen, $NO_2$ or COOH, or $R_4$ and $R_5$ together may represent a group $-OCH_2-O-$;
provided that: (a) when $R_4$ and $R_7$ are each OH, $R_3$, $R_5$ and $R_6$ are each H and one of $R_1$ and $R_2$ is CN, then the other of $R_1$ and $R_2$ cannot be $CONH_2$; and (b) when $R_3$ and $R_7$ are each H, $R_5$ is OH and $R_4$ and $R_6$ are both H or both $C_{1-5}$ alkyl, then $R_1$ is CN and $R_2$ is CN or the group

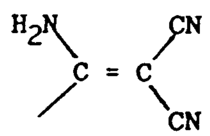 ;

or a pharmaceutically acceptable salt thereof.

**4.** Pharmaceutical compositions according to Claim 3 comprising an active ingredient of formula I in which at one of $R_1$ and $R_2$ is CN cis to the phenyl moiety of said formula, or a pharmaceutically acceptable salt thereof.

**5.** Pharmaceutical compositions according to Claim 4 comprising an active ingredient in which $R_4$ and $R_5$ are hydroxy groups, $R_6$ is hydrogen or hydroxy and $R_3$ and $R_7$ are hydrogens, or a pharmaceutically acceptable salt thereof.

**6.** Pharmaceutical compositions according to Claim 3 containing as an active ingredient a compound selected from:
3,5-dihydroxybenzylidene-malononitrile,
α-hydroxy-(3,4,5- trihydroxybenzylidene)-malononitrile,
3-methoxy-4,5-dihydroxybenzylidene-malononitrile,
α-cyano-3,4-dihydroxycinnamthioamide,
α-cyano-3,4-dihydroxy-cinnamamide,
3,5-di-t-butyl-4-hydroxybenzylidene-malononitrile,
4-formylbenzylidene-malononitrile,
4-hydroxybenzylidene-malononitrile,
3,4-methylenedioxy-6-nitrobenzylidene-malononitrile,
3,4-dihydroxybenzylidene-malonitrile,
3,4,5-trihydroxybenzylidene-malonitrile,
γ-cyano-β-amino-3,4-dihydroxycinnamylidene-malononitrile,
γ-cyano-β-amino-3,4,5-trihydroxycinnamylidene-malononitrile,
γ-cyano-β-amino-3,4-dihydroxy-5-methoxycinnamylidene-malononitrile,
γ-cyano-β-amino-3,4-dihydroxy-5-bromocinnamylidene-malononitrile, and
γ-cyano-β-amino-3-hydroxy-4-nitrocinnamylidene-malononitrile;
and pharmaceutically acceptable salts thereof.

**7.** Novel compounds of formula (I) in Claim 3 and selected from:
3,5-dihydroxybenzylidene-malononitrile,
α-hydroxy-3,4,5-trihydroxybenzylidene-malononitrile,
3-methoxy-4,5-dihydroxybenzylidene-malononitrile,
α-cyano-3,4-dihydroxycinnamthioamide,
4-formylbenzylidene-malononitrile,
3,4-methylenedioxy-6-nitrobenzylidene-malononitrile,

γ-cyano-β-amino-3,4-dihydroxycinnamylidene-malononitrile,
γ-cyano-β-amino-3,4,5-trihydroxycinnamylidene-malononitrile,
γ-cyano-β-amino-3,4-dihydroxy-5-methoxycinnamylidene-malononitrile,
γ-cyano-β-amino-3,4-dihydroxy-5-bromocinnamylidene-malonolnitrile, and
γ-cyano-β-amino-3-hydroxy-4-nitrocinnamylidene-malononitrile;
and pharmaceutically acceptable salts thereof.

**8.** A process for the preparation of 3,5-dihydroxybenzylidene-malononitrile, 3-methoxy-4,5-dihydroxybenzylidene-malononitrile, 3,4,5-trihydroxybenzylidene-malononitrile, 3,4-methylendioxy-6-nitro-benzylidene-malononitrile, γ-cyano-β-amino-3,4-dihydroxy-5-methoxycinnamylidene-malononitrile, and γ-cyano-β-amino-3,4-dihydroxy-5-bromocinnamylidene-malononitrile which comprises reacting the corresponding substituted benzaldehyde with malononitrile in a polar organic solvent and in the presence of a suitable catalyst.

**9.** A process for the preparation of -hydroxy 3,4,5-trihydroxybenzylidene-malononitrile, which comprises reacting triacetyl galloyl chloride with malononitrile in the presence of an amine in a non-polar organic solvent, and hydrolyzing the product.

**10.** A process for the preparation of γ-cyano-β-amino-3,4-dihydroxycinnamylidene-malononitrile, γ-cyano-β-amino-3,4,5-trihydroxycinnamylidene-malononitrile and γ-cyano-β-amino-3-hydroxy-4-nitrocinnamylidene-malononitrile, which comprises reacting 3,4-dihydroxybenzaldehyde with malononitrile dimer in a polar organic solvent and in the presence of a suitable catalyst.

**11.** A process for the preparation of α-cyano-3,4-dihydroxycinnamthioamide which comprises reacting 3,4-dihydroxybenzaldehyde with cyanothioacetamide in the presence of a suitable catalyst.

**12.** The use of compounds of formula (I) as defined in Claim 3 as specific protein-tyrosine kinase inhibitors.

FIGURE 1

INHIBITION OF EGF RECEPTOR KINASE BY ARYLIDENE DERIVATIVES

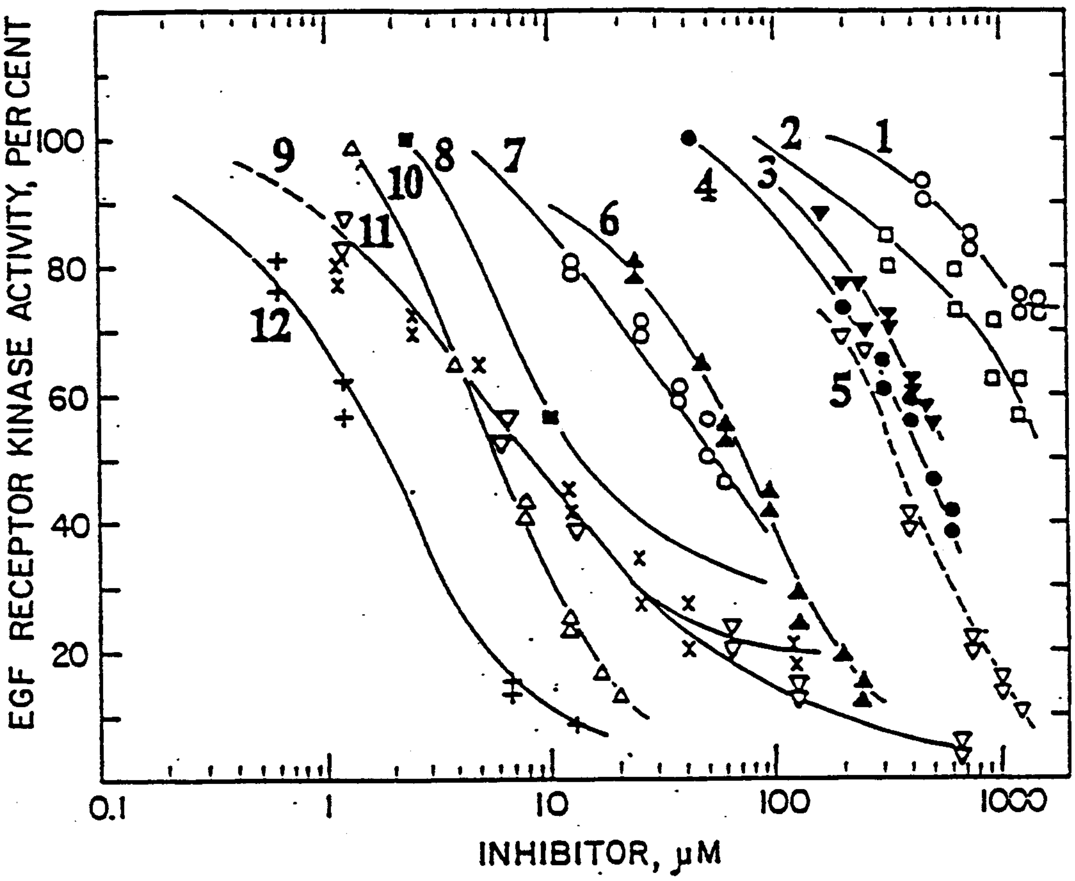


| COMPOUND | SUBSTITUENTS $R_3$ | $R_4$ | $R_5$ | $R_6$ | $R_7$ | $R_2$ | $R_1$ | KInh µM |
|---|---|---|---|---|---|---|---|---|
| 1 | H | H | OH | H | H | $CO_2H$ | H | 1000 |
| 2 | H | H | OH | H | H | $CO_2H$ | $CO_2H$ | 500 |
| 3 | H | H | OH | H | H | CN | CN | 166 |
| 4 | H | OH | OH | H | H | $CO_2H$ | H | 150 |
| 5 | H | H | H | OH | H | CN | CN | 123 |
| 6 | H | OH | H | H | OH | CN | $CO_2H$ | 24 |
| 7 | H | H | OH | OH | H | $CO_2H$ | CN | 18 |
| 8 | H | H | OH | OH | H | CN | CN | 11 |
| 9 | H | $OCH_3$ | OH | OH | H | CN | CN | 2 |
| 10 | H | OH | OH | OH | H | CN | CN | 1 |
| 11 | H | H | OH | OH | H | $CONH_2$ | CN | 2.3 |
| 12 | H | H | OH | OH | H | $CSNH_2$ | CN | 0.85 |

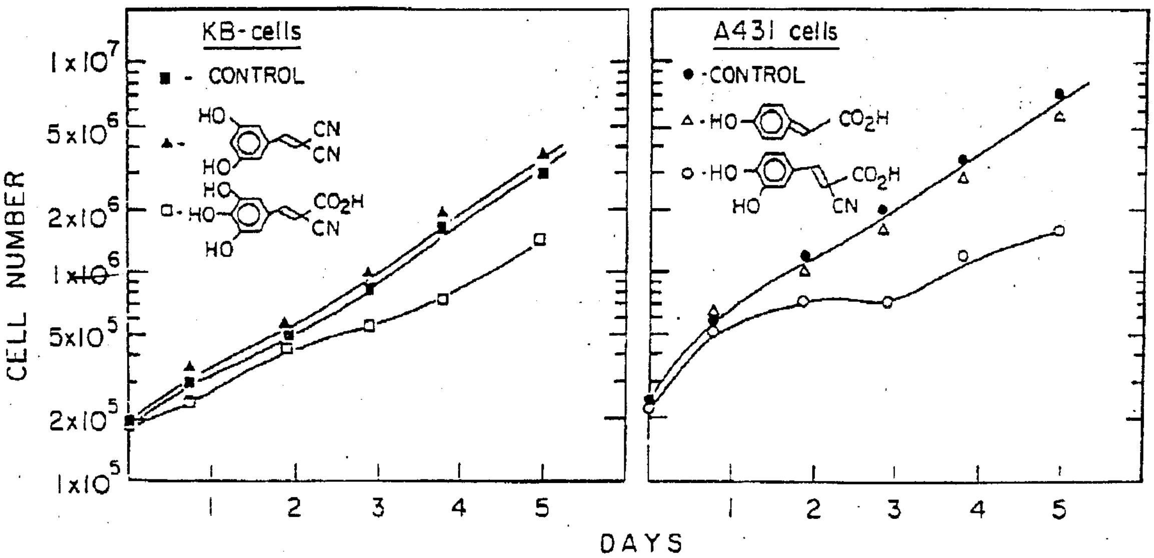
KB-cells
CONTROL
A431 cells
CONTROL
CELL NUMBER
1x10^7
5x10^6
2x10^6
1x10^6
5x10^5
2x10^5
1x10^5
1
2
3
4
5
DAYS

2a

2b

Fig. 2

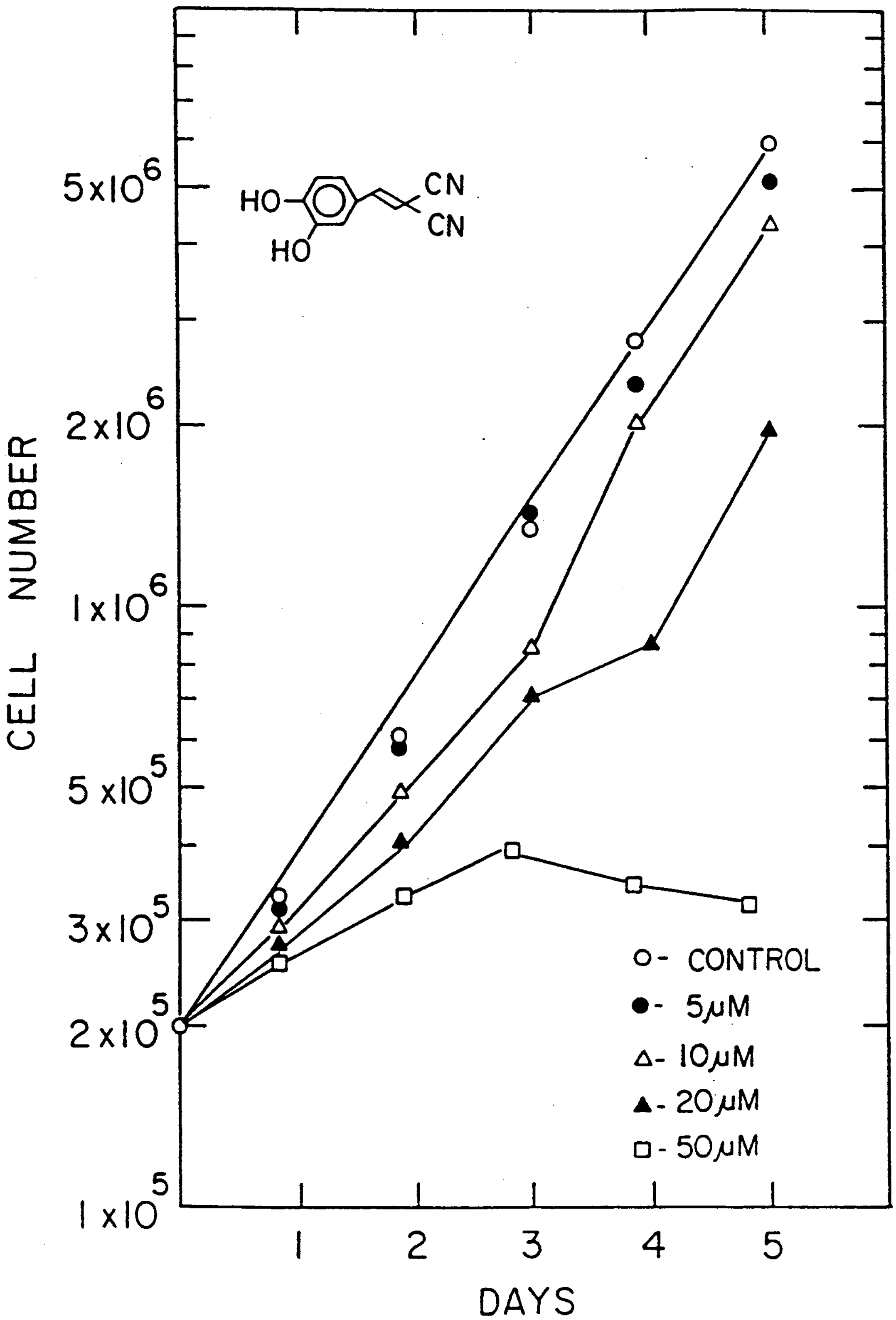
CELL NUMBER
5x10^6
2x10^6
1x10^6
5 x10^5
3x10^5
2x10^5
1 x10^5
1
2
3
4
5
DAYS
○- CONTROL
●- 5μM
△- 10μM
▲- 20μM
□- 50μM

Fig. 3

FIG. 4a

FIG. 4b

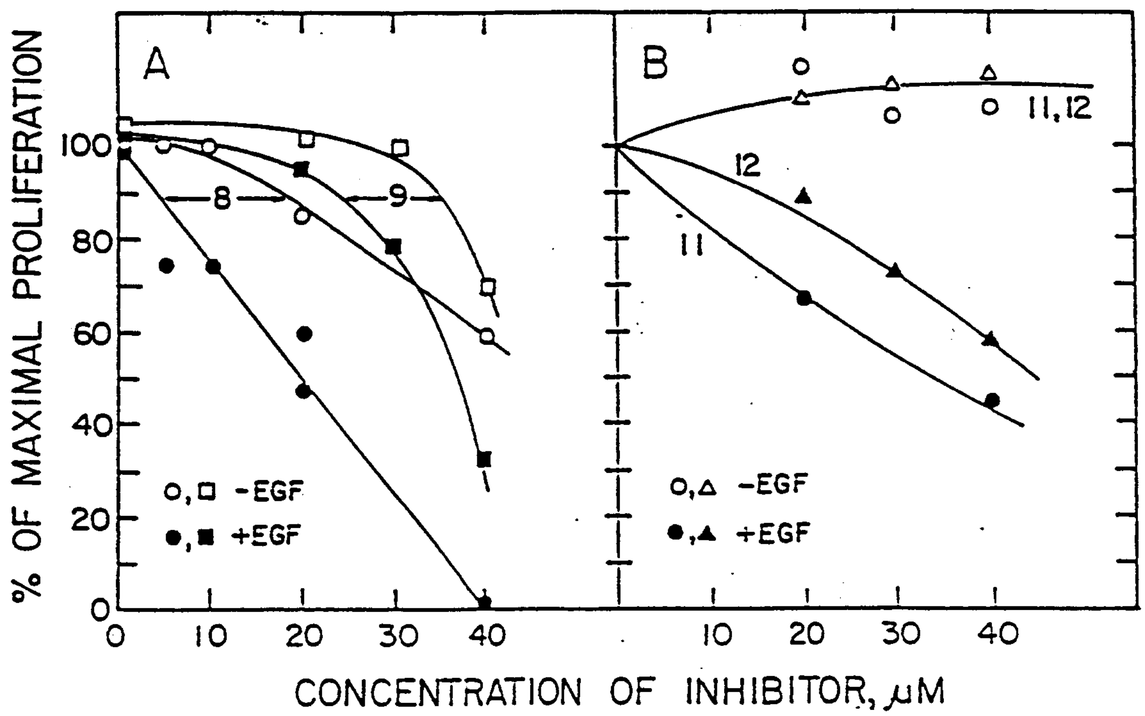
A
B
% OF MAXIMAL PROLIFERATION
100
80
60
40
20
0
8
9
11
12
11,12
○,□ -EGF
●,■ +EGF
○,△ -EGF
●,▲ +EGF
0
10
20
30
40
10
20
30
40
CONCENTRATION OF INHIBITOR, μM